Europäisches Patentamt

European Patent Office

Office européen des brevets

(11) Publication number : **0 505 146 A1**

# EUROPEAN PATENT APPLICATION

(21) Application number : **92302286.7**

(22) Date of filing : **17.03.92**

(51) Int. Cl.⁵ : **C07C 37/80, C07C 39/04**

(30) Priority : **19.03.91 US 671558**

(43) Date of publication of application :
**23.09.92 Bulletin 92/39**

(84) Designated Contracting States :
**DE ES FR GB IT NL**

(71) Applicant : **GENERAL ELECTRIC COMPANY**
**1 River Road**
**Schenectady, NY 12345 (US)**

(72) Inventor : **Fulmer, John William**
**78 Park Ridge Drive**
**Mount Vernon, Indiana 47620 (US)**
Inventor : **Kight, William Dale**
**Box 274**
**Poseyville, Indiana 47633 (US)**

(74) Representative : **Pratt, Richard Wilson et al**
**London Patent Operation G.E. Technical**
**Services Co. Inc. Essex House 12/13 Essex**
**Street**
**London WC2R 3AA (GB)**

(54) **Process for purifying phenol.**

㊼ In a process for purifying phenol which comprises introducing into a distillation tower, a liquid phenol composition comprising above about 95 weight percent phenol on a dry basis and organic contaminants, and introducing fresh water into the distillation tower in order to reduce certain of the contaminants in a hydroextraction process, the improvement comprising adding at least a portion of said fresh water into the distillation tower at or near the bottom of the hydroextractive zone whereby such water removes the organic contaminants through a countercurrent process of contacting the phenol composition.

EP 0 505 146 A1

## BACKGROUND OF THE INVENTION

Phenol and acetone have been mutually prepared by the cumene basic process for almost forty (40) years as disclosed in U.S.P. 4,567,304. Cumene is peroxidized to give cumene hydroperoxide which is then acid cleaved to provide phenol and acetone. The phenol and acetone are then split by a distillation column via their separate boiling points followed by various purification procedures for both the phenol and acetone. Obviously byproducts are formed as well as the desired products of phenol and acetone. Particularly detrimental to phenol quality are color embodying impurities generally of the carbonyl family. Of particular concern are the alphahydroxycarbonyls as typified by the main contaminant, alpha-hydroxyacetone. These particular compounds are deleterious to the quality of phenol, particularly in the coloring of end products from phenol. It is therefore very important to remove as much of these carbonyl contaminants from the phenol stream as possible as well as any other contaminants.

The organic carbonyl contaminants as well as other contaminants such as C4-C6 alkylbenzenes, 2 methyl-benzofuran and the like flow through the phenol purification scheme and are present in the distillation tower in the phenol purification train wherein a hydroextractive distillation occurs. The phenol stream is usually obtained from a prior distillation tower wherein the phenol stream is separated as overhead from the heavy ends. Water is employed so that under the temperature present in the distillation tower the hydroextraction process occurs wherein contaminant oils present in the phenol stream are removed from the phenol as an azeotrope in the overhead. Examples of such "oils" include 2-methylbenzofuran, alphamethylstyrene, 2-phenyl-butene and $C_6$-$C_9$ ketones, such as 3- methylcyclopentenone. Although some of these "oils" may have boiling points significantly different than phenol, they have an affinity and a tendency to group together with phenol in the distillation towers. Generally also present in the tower, although not necessarily desired, is a water/phenol azeotrope which is also removed.

This occurs in the upper portion of the tower. Below, in the dehydration zone of the tower, the section occurring below the acids draws: see Fulmer USP 4,832,796, incorporated by reference into herein, wherein organic acids are withdrawn after reaching a certain pH, residual water is stripped upward and dry phenol is recovered from the bottoms in the tower.

As shown in USP 4,832,796 and generally utilized throughout the art, the water which is utilized for the azeotropic distillations is added to the tower with the phenol or alone but at the top of the tower or near top of the tower as shown in the drawing accompanying USP 4,832,796.

It is now been found that the removal of contaminants in general can be improved and facilitated by introducing at least a portion of the extraneous fresh water employed in the hydroextractive zone of the tower disclosed in USP 4,832,796 at the side of the tower at a point just above the dehydration zone and the acid draws. This corresponds to the bottom of the hydroextractive zone. Adding water at this side point allows a counter current extraction process to occur wherein the upflowing fresh water (steam) contacts downflowing phenol where it is leanest in impurities. This provides the optimum condition for extraction efficiency.

By utilizing the aforesaid new entry point for the water, improvements are gained in phenol product purity. The total amount of water employed need not be increased over that presently used in the standard art. Thus the improvements in phenol quality can be achieved without any cost increase, additional energy or waste water disposal. Additionally, operating efficiency can be increased and investment cost reduced.

## SUMMARY OF THE INVENTION

In a process for purifying phenol which comprises introducing into a distillation tower, a liquid phenol composition comprising above about 95 weight percent phenol on a dry basis and organic contaminants, and introducing fresh water into the distillation tower in order to reduce certain of the contaminants in a hydroextraction process, the improvement comprising adding at least a portion of said fresh water into the distillation tower at or near the bottom of the hydroextractive zone whereby such water removes the organic contaminants through a countercurrent process of contacting the phenol composition.

In accordance with the invention there is a process for purifying phenol which comprises

a. introducing into a distillation tower a liquid phenol composition comprising above about 95 weight percent phenol on a dry basis and organic contaminants,

b. introducing fresh water into the distillation tower in order to reduce organic contaminants in phenol wherein at least a portion of the fresh water is introduced into the tower at a point at or near the bottom of the hydroextractive zone, and

c. recovering purified phenol as bottoms from the distillation tower.

In this manner, substantially more organic contaminants are removed from the phenol composition than when the fresh water is introduced into the column in the art accepted position - near or at the top of the tower.

Not only is a higher purity phenol obtained but when the fresh water is preferably introduced into the distillation tower as steam, lower operating costs (steam usage) and lower capital investment, elimination of a heat exchanger, may also occur.

## DETAILED DESCRIPTION OF THE INVENTION

A phenol composition is introduced into this distillation column for further purification. Generally, although not intended to be restrictive, the phenol is above about 95 wt.% pure on a dry basis, preferably above about 97 wt.%. The remainder comprises almost totally organic contaminants such as for example alphamethyl-styrene, $C_4$ to $C_6$ alkyl benzenes, mesityl oxide, hydroxyacetone, 2-methylbenzofuran, $C_3/C_7$ carbonyls, cumene, acetone, acetophenone and the like. In order to remove the organic contaminants in the aforementioned hydroextractive process for example as found in Fulmer, USP 4,832,796, incorporated by reference herein, fresh water is introduced into the tower. As mentioned, until this invention, this fresh water had been introduced into the tower at or near the top of the tower together with or apart from the phenol composition being purified.

We have now found that a far more complete removal of contaminants occurs when at least a portion of the fresh water enters the column at a point which is at or near the bottom of the hydroextractive zone. By the hydroextractive zone is meant the portion of the tower which has sufficient water therein to extract organic contaminants from the phenol composition. Generally this portion of the tower starts just above where the side draws remove the organic acid contaminants. This is shown in Fulmer USP 4,832,796 in the Figure at 16 wherein the strongly acidic organic acid contaminants are withdrawn. If the tower is operated according to the prior art before Fulmer USP 4,832,796, the hydroextractive zone begins above the acid draws in that process. The preferred introduction point for the water is immediately above the acid draws. However, the introduction point for the water into the tower may be as high up the tower as possible as long as one would still see significant reduction in organic contaminants compared to the contaminants observed with the present water introduction points. Generally the introduction point is no higher than about halfway between the acid draws and phenol introduction, preferably no more than about one quarter higher.

At least a portion of the fresh water introduced into the column is introduced at the invention point. Enough fresh water is introduced here to bring about a significant reduction in contaminants in comparison to contaminants remaining after the prior art complete introduction of water in the high portion or near the top of the tower. Generally, a minimum of about 25% of the fresh water should be introduced at the invention point. Preferably about 40% or more fresh water is introduced. Most preferably 100% of the fresh water enters the tower at the invention point.

The preferred method of introducing the fresh water is in the gaseous phase, i.e., steam. Steam in the range of about 250-365°F is preferred. A pressure of about 20-190 psi is also preferred. By use of steam at such a temperature and pressure, substantial savings in operating costs over the introduction of water in its liquid state can be made. Additionally investment costs may be significantly reduced since an additional heat exchanger may be omitted.

Although stream flow to the tower can vary significantly, it is preferred to maintain a ratio of fresh water feed charge to phenol feed charge of from about 0.03-0.18:1, more preferably 0.05-0.15:1. The units are in volume per time e.g. gallons per minute. Most preferably a ratio of about 0.05 can be used.

It should be noted that there is also a side draw which completes the circulation loop for water, organic contaminants and higher boiling materials such as phenol. This draw is located within and preferably at the bottom below the water inlet of the hydroextraction zone. This circulating loop with the assistance of reboilers therein provides additional heat to the distillation tower. This loop provides a convenient point for introduction of the fresh water (steam). For example in a 65 tray distillation tower, the acid draws could be at trays 48-49 (from the top) while at least a portion of the fresh water is introduced at tray 45 wherein it moves upward in a countercurrent to the phenol stream manner. An outlet for water and organic contaminants could be located at tray 46. Such side draw would form a closed system for recycle of the water and contaminants.

In phenol distillation towers involving the use of water in a hydroextractive process, the light ends, contaminants, exit the tower at the top (overhead) while purified phenol is obtained at the bottoms draw. The light ends overhead, comprised of at least water, carbonyls, 2-methylbenzofuran, alpha methylstyrene, $C_4$-$C_6$ alkyl-benzenes and the like, are outletted to a reflux drum where the top organic layer is purged off frequently while the bottom layer, water with organic contaminants therein, is sent back to the tower as aqueous reflux. Generally, the ratio of stream flows of aqueous reflux: phenol feed is about 1.0-2.5:1, preferably about 1.2-2.2:1 in units of volume per time. It is most preferred to use a reflux to phenol feed ratio of about 1.2:1.

As stated before, below the acid draws are located just above the "dehydration" portion of the tower. Within this zone, the temperature is raised steadily so as to remove any water left over and separate phenol from higher boiling impurities.

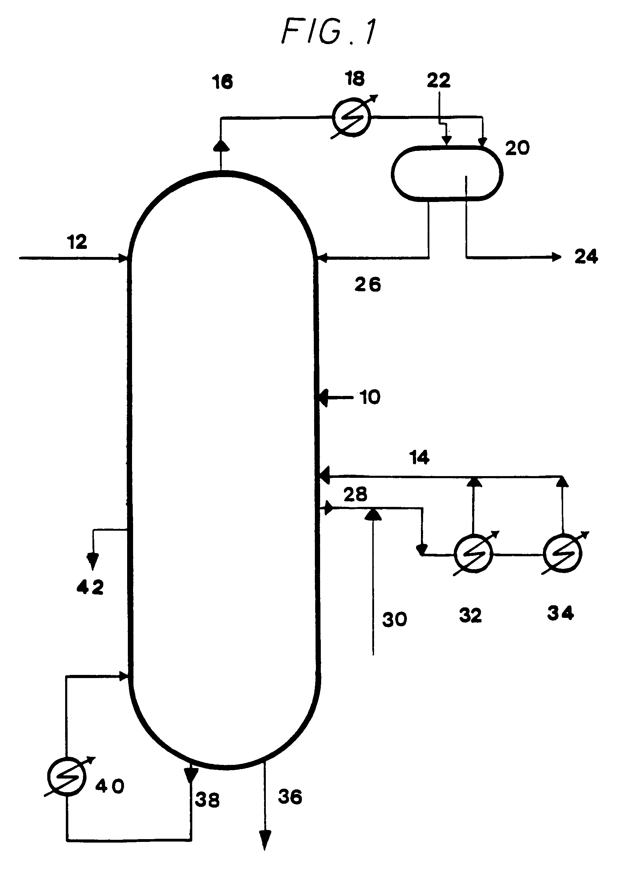

Figure 1 - a figure showing a process invention embodiment.

The process is now explained in relation to Figure 1. Into a distillation tower, 10, is introduced a phenol stream, 12, at the rate of about 185 gallons per minute (gpm). The phenol is approximately 97 wt% phenol, as measured in the dry state. As the phenol composition makes its way down the 65 tray tower into steadily increasing temperature zones, it is met by water entering into the tower through line, 14, at tray 45. This water moves upward in a countercurrent processing mode and extracts contaminants from the downwardly flowing phenol stream thereby leaving the tower as water, an azeotrope with contaminants of the phenol stream and/or phenol, and the like at line, 16. This aqueous mass goes through a heat exchanger, 18 and enters the reflux drum, 20. At this point additional fresh water may enter reflux drum, 20, through line, 22. In the reflux drum, the aqueous mass separates into two layers, a top primarily organic layer and a bottom primarily aqueous layer. The organic layer overflows and is withdrawn as purge through line, 24, at a rate of 17 gpm. The aqueous layer is returned to the upper portion of the tower through line 26. Additionally, an aqueous portion is removed from the tower at tray 46 through line 28. Fresh water is added at a rate of 12 gpm to this line at line 30. The aqueous mass goes through several heat exchangers 32 and 34 and is returned to the tower through previously mentioned line, 14. The purified phenol composition, 99.9+ wt.% phenol leaves the tower at the bottom ,36, at a rate of 180 gpm. Heating of the tower is done through withdrawing material through line, 38, passing through heat exchanger (reboiler) 40 and returning the heated mass to the tower. Therefore a volume balance has also been shown. 185 gpm phenol + 12 gpm fresh water entering the tower = 180 gpm purified phenol +17 gpm purge leaving the tower. The amount of acid taken out by the draws, 42, at trays 48 and 49 is quite small on a minute basis.

EXAMPLE

In order to prove the concept of reduced contaminants, a plant run was made against the prior art control. In the prior art run 100% of the fresh water was added at the top, for example through line, 22 of the Figure. In the run according to the invention, Example 1, only 50% of the fresh water was added at the top. The other 50% was added at the end of the hydroextractive zone, see Figure line 30. The water was added as water, not as steam. The control experiment water was added at a rate of 20 gpm. Example 1 water was added at a rate of 10 gpm top and 10 gpm side. The reflux: phenol feed ratio was 1.6:1 in both cases and the feed rate of the phenol feed stock stream was 185 gpm in both cases.

The feed composition was the following:

| Material | wt % |
|---|---|
| Phenol | 95-97 |
| alpha methylstyrene | 0.5-2.5 |
| C4/C6 alkylbenzenes | 0.5-2.0 |
| Mesityl oxide | 0.1-0.3 |
| Water | 0.01-0.1 |
| | |
| Hydroxyacetone | 10-200 ppm |
| 2-methylbenzofuran | 200-800 ppm |
| C3/C7 carbonyls | 50-200 ppm |
| Cumene | 50-100 ppm |
| Acetone | 50-200 ppm |
| Acetophenone | 10-20 ppm |

The phenol bottoms in both the control and Example 1 were analyzed for impurities by gas phase chromatography and other methods. Below are the results.

|                        | <u>Control</u>, ppm | <u>Example 1</u>, ppm |
|------------------------|---------------------|-----------------------|
| Total gc Impurities    | 100-116             | 30-50                 |
| 2-methylbenzofuran     | 10-15               | 3-4                   |
| $C_3$-$C_6$ ketone     | 60-70               | 10-15                 |
| Hydroxyacetone         | 10-20               | 10-20                 |
| Total carbonyls (wet)  | 80-90               | 30-40                 |

As is readily observed, the use of the inventive process results in a significant reduction of contaminants.

**Claims**

1. In a process for purifying phenol which comprises introducing into a distillation tower, a liquid phenol composition comprising above about 95 weight percent phenol on a dry basis and organic contaminants, introducing fresh water into the distillation tower in order to reduce certain of the contaminants in a hydroextraction process, the improvement comprising adding at least a portion of said fresh water into the distillation tower at or near the bottom of the hydroextractive zone whereby such water removes the organic contaminants through a countercurrent process of contacting the phenol composition.

2. The process of claim 1 wherein the fresh water added at or near the bottom of the hydroextractive zone is at least about 25% of the fresh water.

3. The process of claim 2 wherein the fresh water is added as steam.

4. The process of claim 3 wherein all the fresh water is added at or near the bottom of the hydroextractive zone.

5. The process of claim 1 wherein fresh water added to the tower as added on the basis of a volumetric ratio of from about 0.03-0.18:1, based on the rate of phenol composition addition.

6. A process for purifying phenol which comprises
   a. introducing into a distillation tower a liquid phenol composition comprising above about 95 weight percent phenol on a dry basis and organic contaminants,
   b. introducing fresh water into the distillation tower in order to reduce organic contaminants in phenol wherein at least a portion of the fresh water is introduced into the tower at a point at or near the bottom of the hydroextractive zone, and
   c. recovering purified phenol as bottoms from the distillation tower.

7. The process of claim 6 wherein the fresh water added at or near the bottom of the hydroextractive zone is at least about 25% of the fresh water.

8. The process of claim 7 wherein the fresh water is added as steam.

9. The process of claim 8 wherein all the steam is added at or near the bottom of the hydroextractive zone.

10. The process of claim 6 wherein fresh water added to the tower is added at the volumetric ratio from about 0.03-0.18:1, based on the rate of the phenol composition addition.

# FIG.1

EP 0 505 146 A1

**European Patent Office**

# EUROPEAN SEARCH REPORT

Application Number

EP 92 30 2286

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 299 239 (GENERAL ELECTRIC COMPANY)<br>* page 5, line 3 - line 38 *<br>--- | 1-4,6-9 | C07C37/80<br>C07C39/04 |
| Y | GB-A-753 475 (CALIFORNIA RESEARCH CORPORATION)<br>* the whole document *<br>--- | 1-4,6-9 | |
| A | US-A-4 857 151 (G.D. SUCIU ET AL.)<br>* the whole document; especially the figure and column 4, line 66 - column 5, line 7 *<br>--- | 1-10 | |
| A | US-A-2 992 169 (G.P. GREGORY ET AL.)<br>* column 8; claims 1,2 *<br>* column 3 - column 4; example 1 *<br>* figure 1 *<br><br>----- | 1-10 | |
| | | | **TECHNICAL FIELDS SEARCHED (Int. Cl.5)**<br><br>C07C |
| | The present search report has been drawn up for all claims | | |

| Place of search | Date of completion of the search | Examiner |
|---|---|---|
| THE HAGUE | 29 JUNE 1992 | FINK D.G. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.........................................................................
& : member of the same patent family, corresponding
   document

EPO FORM 1503 03.82 (P0401)

7